# EUROPEAN PATENT APPLICATION

(11) **EP 2 116 844 A1**
(43) Date of publication of application: **11.11.2009**
(21) Application number: 08703731.3
(22) Date of filing: 23.01.2008
(51) Int. Cl.: G01N 27/26, G01N 27/416

(54) **CONTROL LIQUID JUDGING METHOD AND ANALYSIS DEVICE**

(30) Priority: 23.01.2007 JP 2007013155
(71) Applicant: Arkray, Inc., Kyoto-shi, Kyoto 601-8045 (JP)
(72) Inventor: MATSUDA, Hirokazu, Kyoto-shi Kyoto 601-8045 (JP); SATO, Yoshiharu, Kyoto-shi Kyoto 601-8045 (JP)
(74) Representative: HOFFMANN EITLE
(86) International application number: PCT/JP2008/050901
(87) International publication number: WO 2008/090925

(57) **Abstract**

The present invention relates to a method for distinguishing between a sample and a control liquid in a system for analyzing a specific component in the sample by using an analyzing tool having a working electrode and an counter electrode. This discriminating method includes a first step of applying a voltage between the working electrode and the counter electrode, a second step of measuring a response current at certain intervals by use of the working electrode and the counter electrode, a third step of calculating a relative value for a peak value or an end value of the response current, a fourth step of calculating a change rate of the relative value, and a fifth step of distinguishing between the sample and the control liquid based on the change rate.

## Description

### TECHNICAL FIELD

The present invention relates to a method for distinguishing between a sample and a control liquid in an analyzing system for analyzing a specific component in the sample.

### BACKGROUND ART

Knowing biological information such as a concentration of glucose in blood is important for finding and treating various diseases. Examples of a method of obtaining the biological information in blood include a method of using an analyzing tool such as a biosensor or the like. In this method, a blood sample is supplied to a reaction reagent layer disposed on the analyzing tool to react the blood sample with a reagent, and based on a reaction product at this time, information according to a concentration of a specific component in the blood sample is detected in a concentration measurement apparatus using an electrochemical technique or an optical technique.

In such a concentration measurement apparatus, in order to secure reliability of measurement result, it is necessary to inspect the apparatus to see whether the apparatus functions normally or not periodically or when the apparatus was not used for a long period of time. Generally, in the inspection of the concentration measurement apparatus, a user operates the concentration measurement apparatus and selects a control liquid measurement mode manually, and mounts an analyzing tool on the apparatus and supplies a control liquid to the analyzing tool, and thereby the inspection is performed.

In such a method, a user not only needs an operation for performing the operational inspection of an apparatus but also needs an operation for returning to a normal measurement mode after the completion of apparatus inspection and this is a large burden. Moreover, there arises a situation where the inspection of the apparatus is performed without changing a mode from a normal measurement mode to a control liquid measurement mode, and conversely the measurement of the sample is performed without changing a mode from a control liquid measurement mode to a normal measurement mode. As a result, problems that accurate inspection results or measurement results cannot be obtained and the need for reinspecting or remeasuring arise.

In order to solve such problems, there are proposed techniques in which a control liquid is automatically recognized in a concentration measurement apparatus to perform an inspection of the apparatus (for example, refer to Patent Documents 1 to 3).

A method described in Patent Document 1 is a method which focuses on a difference in solubility of a reaction reagent layer between a whole blood and a control liquid, and the whole blood is distinguished from the control liquid based on a difference in measured current value between the whole blood and the control liquid.

Patent Document 2 discloses, similar to Patent Document 1, a method for distinguishing between a whole blood and a control liquid based on a difference between measured current values in a measurement system using an electrochemical technique.

A method described in Patent Document 3 is a method in which in the measurement system using the electrochemical technique, a detection electrode is provided in addition to the working electrode and the counter electrode for an electrode type biosensor and a control liquid is automatically distinguished from an oxidation current obtained by using the detection electrode. The method in the above Patent Document is a method focusing on a fact that a behavior of an oxidation current obtained by a reaction of the control liquid with a reaction reagent layer of the biosensor is different from the behavior of an oxidation current obtained by a reaction of the sample with the reaction reagent layer, and a method for automatically distinguishing between the sample and the control liquid based on an oxidation current value at a lapse of a specific time or a change with time of the oxidation current value.

However, the methods in Patent Documents 1 to 3 distinguish between the control liquid and the sample from change in response currents with time. Therefore, it is difficult to distinguish the control liquids of a plurality of concentrations from the samples of various concentrations. Particularly when glucose in the blood sample is measured, it is difficult to distinguish the blood samples having various concentrations and hematocrit levels from the control liquid since the response current is affected by the hematocrit levels.

Patent Document 1: Japanese Unexamined Patent Publication No. 2003-114214
Patent Document 2: Japanese Unexamined Patent Publication No. 2005-531760
Patent Document 3: Japanese Unexamined Patent Publication No. 2001-208718

### DISCLOSURE OF THE INVENTION

### PROBLEMS TO BE SOLVED BY THE INVENTION

It is an object of the present invention to provide a method which enables to automatically discriminate a control liquid to reduce a burden of a measurer and enables to suppress an occurrence of false measurement and accurately discriminate the control liquid.

### MEANS FOR SOLVING THE PROBLEMS

In a first aspect of the present invention, there is provided a method for distinguishing between a sample and a control liquid in a system for analyzing a specific component in the sample by using an analyzing tool having a working electrode and an counter electrode, wherein the method includes: a first step of applying a voltage between the working electrode and the counter electrode; a second step of measuring a response current at certain intervals by use of the working electrode and the counter electrode; a third step of calculating a relative value for a peak value or an end value of the response current; a fourth step of calculating a change rate of the relative value; and a fifth step of distinguishing between the sample and the control liquid based on the change rate.

In the fifth step, an object is determined to be a sample, for example, when a summed value of change rate values at a plurality of specific times is a certain value or more, and determined to be a control liquid when the summed value of change rate values is smaller than the certain value.

Moreover, in the fifth step, an object is determined to be a sample when a summed value of differences between a change rate value at a first specific time and change rate values at a plurality of second specific times is a certain value or more, and determined to be a control liquid when the summed value of differences is smaller than the certain value.

Moreover, in the fifth step, an object may be determined to be a control liquid when an average value of change rate values at a plurality of specific times is a certain value or more, and determined to be a sample when the average value is smaller than the certain value. In this case, a liquid containing mannose is preferably used as a control liquid.

The first step is performed, for example, by continuously applying a certain voltage between the working electrode and the counter electrode.
In this case, in the third step, a calculation is performed, taking a maximum value of the response current obtained in the second step as a peak value, and a calculation is performed using an end value of the response current obtained in the second step.

The first step may be performed by applying a certain voltage between the working electrode and the counter electrode for a certain period of time, then stopping the application of a voltage for a certain period of time, and further applying a certain voltage between the working electrode and the counter electrode. In this case, in the third step, a calculation is performed using a peak value selected from a plurality of peak values of the response current obtained in the second step, and a calculation is performed using an end value of the response current obtained in the second step.

A whole blood, for example, is used as the sample, and the specific component is glucose.

In a second aspect of the present invention, there is provided an analyzing apparatus for analyzing a specific component in a sample by using an analyzing tool having a working electrode and an counter electrode, wherein the analyzing apparatus includes: voltage application means for applying a voltage between the working electrode and the counter electrode; current measuring means for measuring a response current at certain intervals when a voltage is applied between the working electrode and the counter electrode; calculating means for calculating a relative value for a peak value or an end value of the response current and for calculating a change rate of the relative value; and control means for distinguishing between a sample and a control liquid based on the change rate calculated in the calculating means.

The control means is configured so as to determine an object to be a sample, for example, when a summed value of change rate values at a plurality of specific times is a certain value or more, and determine an object to be a control liquid when the summed value of change rate values is smaller than the certain value.

The control means may be configured so as to determine an object to be a sample when a summed value of differences between a change rate value at a first specific time and change rate values at a plurality of second specific times is a certain value or more, and determine an object to be a control liquid when the summed value of differences is smaller than the certain value.

The control means may be configured so as to determine an object to be a control liquid when an average value of change rate values at a plurality of specific times is a certain value or more, and determine an object to be a sample when the average value is smaller than the certain value.

The control means is configured so as to control the voltage application means in order to continuously apply a certain voltage between the working electrode and the counter electrode.
In this case, the calculating means is preferably configured so as to calculate the change rate, taking a maximum value of the response current measured in the current measuring means as a peak value. The calculating means may be configured so as to calculate the change rate, using an end value of the response current measured in the current measuring means.

The control means may be configured so as to control the voltage application means in order to apply a certain voltage between the working electrode and the counter electrode for a certain period of time, then stop the application of a voltage for a certain period of time, and further apply a certain voltage between the working electrode and the counter electrode.
In this case, the calculating means is preferably configured so as to calculate the change rate using a peak value selected from a plurality of peak values of the response current measured in the current measuring means. The calculating means may be configured so as to calculate the change rate using an end value of the response current measured in the current measuring means.

The analyzing apparatus of the present invention is configured, for example, so as to analyze glucose as the specific component in the whole blood as the sample.

### EFFECTS OF THE INVENTION

In the present invention, it becomes possible to automatically distinguish between a sample such as a whole blood and a control liquid in an analyzing system such as an analyzing apparatus which analyzes a sample using an analyzing tool. Therefore, when measuring the control liquid, a user does not need to select a mode for measuring a control liquid and a burden of the user is reduced. Further, if the control liquid can be automatically distinguished, there does not arise a situation where an inspection of the analyzing apparatus is performed without changing a mode from a normal measurement mode to a control liquid measurement mode, and conversely the measurement of the sample is performed without changing a mode from a control liquid measurement mode to a normal measurement mode. As a result, accurate inspection results or measurement results can be obtained and the need for reinspecting or remeasuring does not arise.

Further, in the method for discriminating a control liquid of the present invention, a distinction between the sample and the control liquid is made based on a change rate of a relative value for a peak value or an end value. Calculating the relative value with a peak value or an end value as a reference value facilitates to identify the difference between the sample response current and the control liquid response current, and calculating the above-mentioned change rate of a relative value enables to appropriately distinguish between the sample response current and the control liquid response current.

Particularly, it becomes possible to appropriately distinguish between a sample and a control liquid from the response current by focusing on a range of time where a difference in change rate between the sample response current and the control liquid response current is relatively large, and performing a certain calculation in the time range and comparing the calculated value with a threshold value.

### BRIEF DESCRIPTION OF THE DRAWINGS

Fig. 1 is an overall perspective view showing an example of an analyzing system to which a discriminating method of the present invention is applied.
Fig. 2 is a perspective view showing an example of a biosensor used in the analyzing system shown in Fig. 1.
Fig. 3 is a sectional view taken along line III-III of Fig. 2.
Fig. 4 is an exploded perspective view of the biosensor shown in Fig. 2.
Fig. 5 is a sectional view taken along line V-V of Fig. 1.
Fig. 6 is a block diagram of the analyzing system shown in Fig. 1.
Fig. 7 is a flow chart for illustrating a control liquid discriminating method of the present invention.
Figs. 8A, 8B and 8C are graphs each showing an example of a voltage application pattern.
Fig. 9 is a graph showing a voltage application pattern in Example 1.
Fig. 10 is a graph showing measurement results of a response current of a control liquid in Example 1.
Figs. 11A, 11B and 11C are graphs each showing measurement results of a response current of a whole blood in Example 1.
Fig. 12A is a graph showing calculation results of change rates of the control liquid using a peak value in Example 1, and Fig. 12B is a graph showing calculation results of change rates of the whole blood using the peak value in Example 1.
Fig. 13A is a graph showing calculation results of change rates of the control liquid using an end value in Example 1, and Fig. 13B is a graph showing calculation results of change rates of the whole blood using the end value in Example 1.
Fig. 14 is a graph showing a voltage application pattern in Example 2.
Fig. 15 is a graph showing measurement results of a response current of a control liquid in Example 2.
Figs. 16A, 16B and 16C are graphs each showing measurement results of a response current of a whole blood in Example 2.
Fig. 17A is a graph showing calculation results of change rates of the control liquid using a peak value in Example 2, and Fig. 17B is a graph showing calculation results of change rates of the whole blood using the peak value in Example 2.
Fig. 18A is a graph showing calculation results of change rates of the control liquid using an end value in Example 2, and Fig. 18B is a graph showing calculation results of change rates of the whole blood using the end value in Example 2.
Fig. 19 is a graph showing a voltage application pattern in Example 3.
Fig. 20 is a graph showing measurement results of the response current of the control liquid in Example 3.
Figs. 21A, 21B and 21C are graphs each showing measurement results of a response current of a whole blood in Example 3.
Fig. 22A is a graph showing calculation results of change rates of the control liquid using an end value in Example 3, and Fig. 22B is a graph showing calculation results of change rates of the whole blood using an end value in Example 3.
Figs. 23A, 23B, and 23C are graphs each showing calculation results of a change rate in Example 4.

### DESCRIPTION OF THE REFERENCE NUMERALS

- 1: Analyzing apparatus
- 11: Power supply (voltage application means)
- 12: Current measuring part (current measuring means)
- 13: Calculating part (calculating means)
- 14: Control part (control means)
- 2: Biosensor (analyzing tool)
- 24: Working electrode
- 25: Counter electrode

### BEST MODE FOR CARRYING OUT THE INVENTION

Hereinafter, the present invention will be described specifically with reference to the drawings.

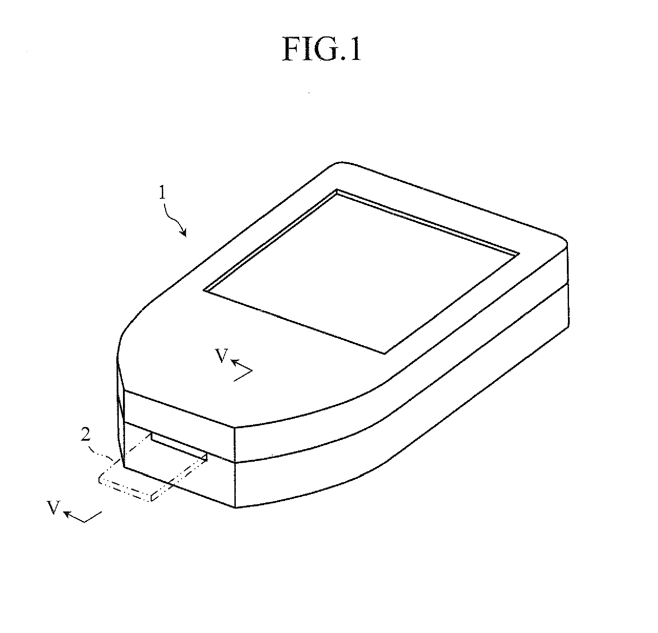

An analyzing apparatus 1 shown in Fig. 1 is configured so as to measure a concentration of a specific component in a sample using a biosensor 2.

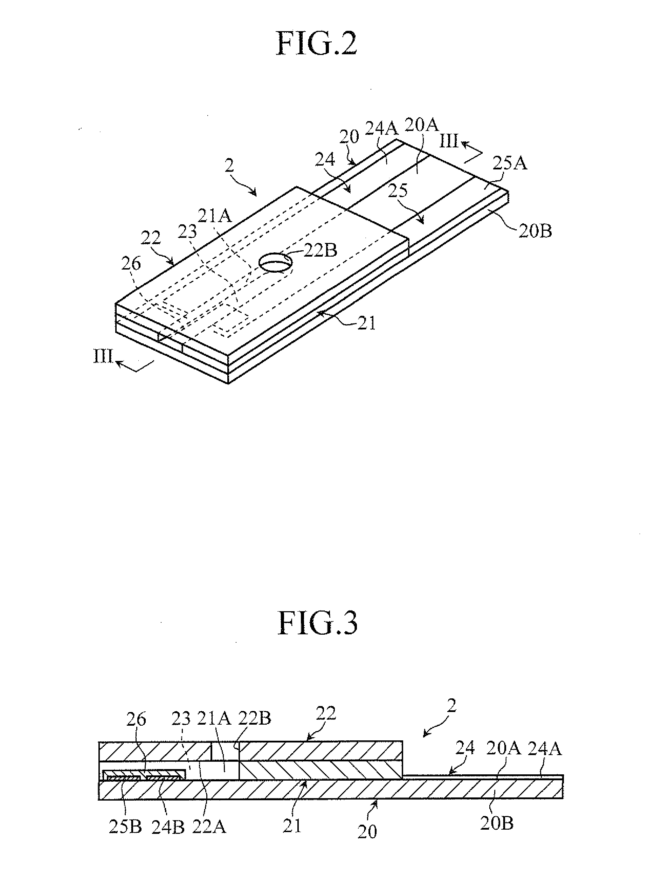

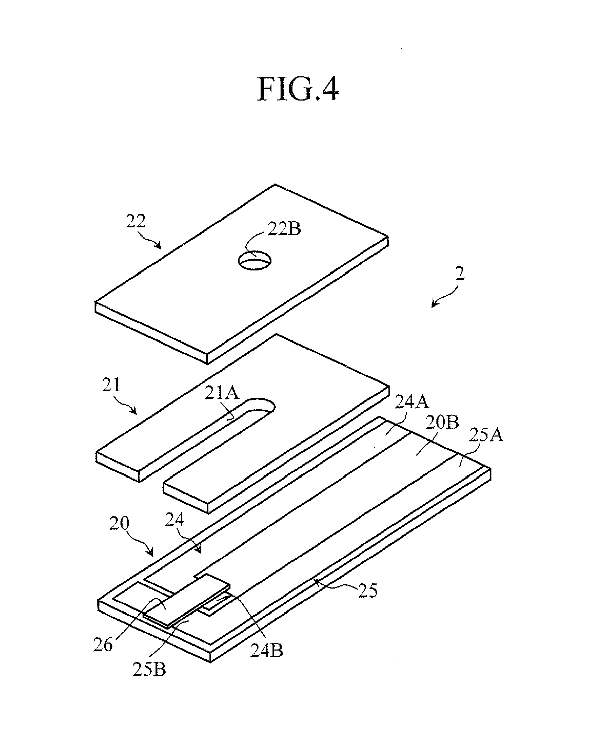

The biosensor 2 is configured so as to be disposable and formed in a flat plate shape as a whole. As shown in Fig. 2 and Fig. 4, the biosensor 2 has a structure in which a cover 22 is joined to an approximately rectangular substrate 20 with a spacer 21 therebetween. In the biosensor 2, a capillary 23 extending in a longitudinal direction of the substrate 20 is defined by the respective constituents 20, 21 and 22.

The spacer 21 is provided for defining a distance between an upper surface 20A of the substrate 20 and a lower surface 22A of the cover 22, namely, a height dimension of the capillary 23, and is composed of a double-faced tape, for example. The spacer 21 is provided with a slit 21A for defining a width dimension of the capillary 23.

The cover 22 has an exhaust port 22B for exhausting a gas within the capillary 23 to the outside. The cover 22 is made of a thermoplastic resin having high wettability such as vinylon, highly crystallized PVA or the like.

The substrate 20 is made of an insulating resin material in a shape larger than the cover 22, and a working electrode 24, an counter electrode 25 and a reagent layer 26 are formed on the upper surface 20A of the substrate 20.

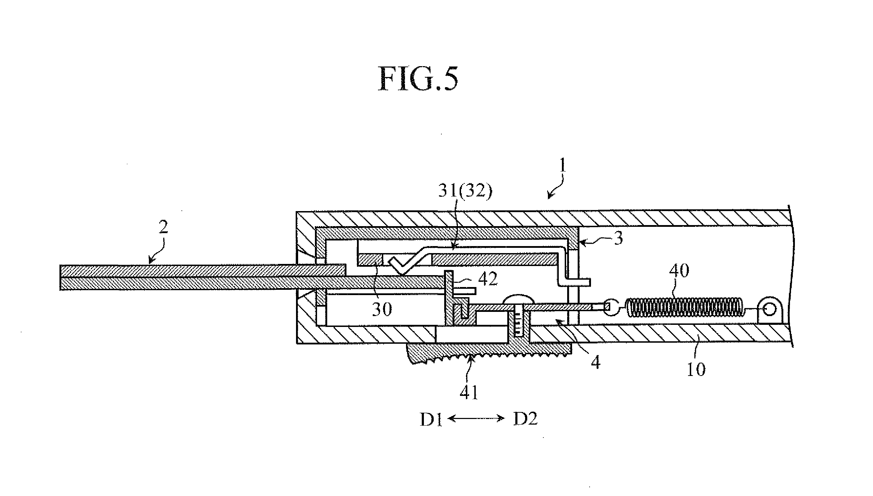

The working electrode 24 and the counter electrode 25 are provided for applying a voltage to blood introduced into the capillary 23, and ends 24A and 25A thereof are exposed without being covered with the cover 22. These ends 24A and 25A are portions which are brought into contact with terminals 31 and 32 (refer to Fig. 5) of a connector part 3 when the biosensor 2 is fitted to the analyzing apparatus 1. The ends 24B and 25B of the working electrode 24 and the counter electrode 25 extend in a width direction of the substrate 20 and parts of the ends 24B and 25B are located at the inside of the capillary 23. The reagent layer 26 is disposed so as to cover the ends 24B and 25B of the working electrode 24 and the counter electrode 25, and located at the inside of the capillary 23. The reagent layer 26 includes, for example, an electron transfer material (complexes such as [Ru(NH₃)₆]Cl₃, K₃[Fe(CN)₆] or the like) and an oxidation-reduction enzyme (glucose oxidase (GOD), glucose dehydrogenase (GDH), or the like), and is formed in a solid form which is readily dissolved in blood.

The capillary 23 is provided for moving a liquid (a sample or a control liquid) toward the exhaust port 22B by using a capillary phenomenon and for holding the introduced liquid. When the liquid is introduced into the capillary 23, the reagent layer 26 is dissolved to produce a liquid phase reaction system which includes the electron transfer material, the oxidation-reduction enzyme and the liquid in the capillary 23.

In this case, a biochemical sample such as blood, urea, or saliva is used as a sample, and examples of a specific component which is an object of analysis in the sample include glucose, cholesterol and lactic acid.

As the control liquid, a liquid which contains a specific component such as glucose and a buffer and in which a concentration of the specific component is known is used. The buffer may be one which has a buffering power in a desired pH range, and for example, benzoic acid salt, and tris- or 2-morpholino ethanesulfonic acid (MES) can be used. As the control liquid, a control liquid containing mannose is preferably used. A concentration of the mannose in the control liquid is, for example, 5 M or less. A thickening agent, antiseptics, or a dye may be added to the control liquid. As the thickening agent, various publicly known substances, for example, polyvinyl alcohol (PVA) or ECHO GUM (xanthan gum) can be used. As the antiseptics, various publicly known substances, for example, isothiazolone can be used. The dye may be a dye which can color the control liquid, and food dyes such as food red No.40, food red No.106 and food blue No.1 can be used.

As shown in Fig. 5, the analyzing apparatus 1 includes a connector part 3 and a disposal mechanism 4.

The connector part 3 is a portion to which the biosensor 2 is fitted and has a structure in which a plurality of terminals 31 and 32 are fixed to a terminal base 30.

A plurality of terminals 31 and 32 are provided for contacting with the working electrode 24 and the counter electrode 25 (refer to Fig. 2 or Fig. 4) of the biosensor 2 when the biosensor 2 is fitted to the connector part 3, applying a voltage between these electrodes 24 and 25, and measuring a current value (resistance value) at this time. A tip portion of each of the terminals 31 and 32 is configured in a form of a plate spring and this structure also has a function of holding the biosensor 2 properly at the connector part 3 when the biosensor 2 is fitted to the connector part 3.

The disposal mechanism 4 is provided for disposing the used biosensor 2 from the analyzing apparatus 1. The disposal mechanism 4 has an operation lever 41 biased by a coil spring 40.

The operation lever 41 is a portion which is operated in order to move a press body 42 to extrude the biosensor 2 and is movable to and from in D1 and D2 directions relative to a housing 10 with a part of the operation lever 41 exposed outside the housing 10.

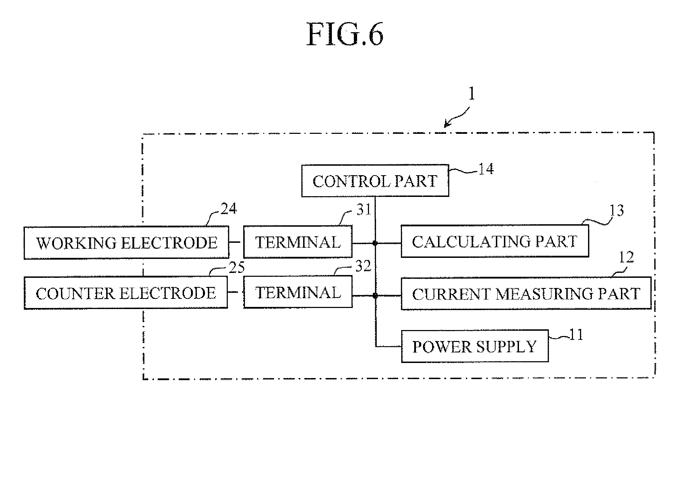

As shown in Fig. 6, the analyzing apparatus 1 further includes a power supply 11, a current measuring part 12, a calculating part 13 and a control part 14.

The power supply 11 is provided for applying a voltage between the working electrode 24 and the counter electrode 25 of the biosensor 2 and is configured, for example, by a DC power supply.

The current measuring part 12 is provided for measuring an amount of electron transfers between the working electrode 24 and a specific component in the sample at the time of applying a voltage between the working electrode 24 and the counter electrode 25.

The calculating part 13 is provided for calculating a concentration of the specific component in the sample based on measurement results in the current measuring part 12 or for performing necessary calculation in distinguishing whether a liquid adhering to the biosensor 2 as a spot is a sample or a control liquid.

The control part 14 controls various operations such as on/off control of a power supply 11, control of measuring timing at the current measuring part 12 and an operation of the calculating part 13.

Next, one example of an operation of the analyzing apparatus 1 will be described.

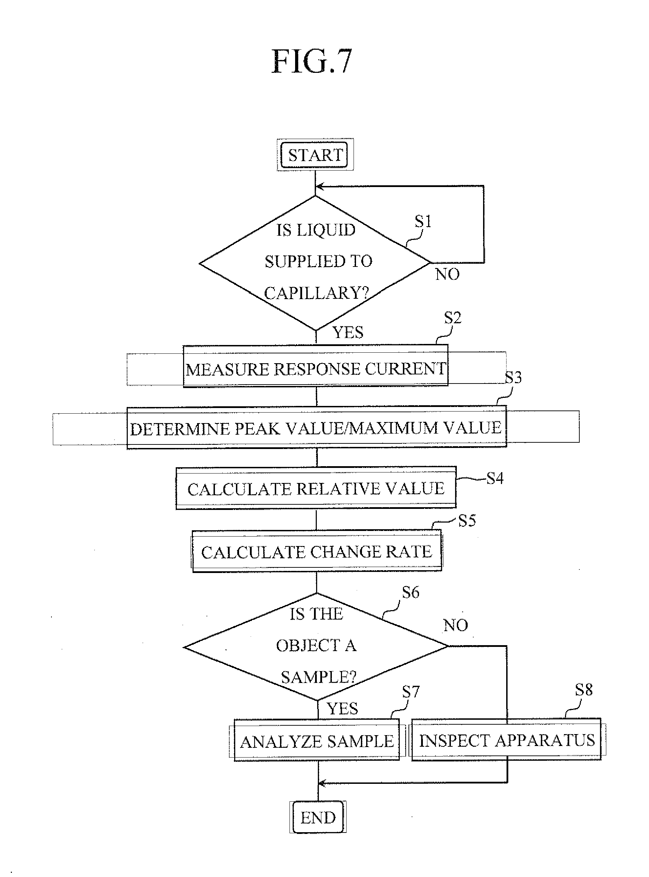

As shown in a flow chart in Fig. 7, the analyzing apparatus 1 determines whether a liquid is supplied to a capillary 23 of the biosensor 2 or not when the biosensor 2 is fitted (S1). This determination is performed by detecting whether in the biosensor 2, the working electrode 24 is communicated with the counter electrode 25 through liquid or not. That is, when a liquid is supplied to the biosensor 2, the capillary 23 of the biosensor 2 is filled with the liquid through a capillary force generated in the capillary 23. Therefore, if a voltage is applied between the working electrode 24 and the counter electrode 25 by the power supply 11, a current flows between the working electrode 24 and the counter electrode 25, and therefore it is possible to detect whether the working electrode 24 is communicated with the counter electrode 25 through liquid or not.

When the analyzing apparatus 1 determines that a liquid is supplied to the biosensor 2 (S1: Yes), the control part 14 distinguishes whether the liquid supplied to the biosensor 2 is a sample or a control liquid (S2 to S6).

First, in a state in which a voltage is applied between the working electrode 24 and the counter electrode 25 by the power supply 11, the current measuring part 12 measures a response current at certain intervals through the working electrode 24 and the counter electrode 25 (S2). A time interval at which the response current is measured is selected from a range of 0.01 seconds to 1 second.

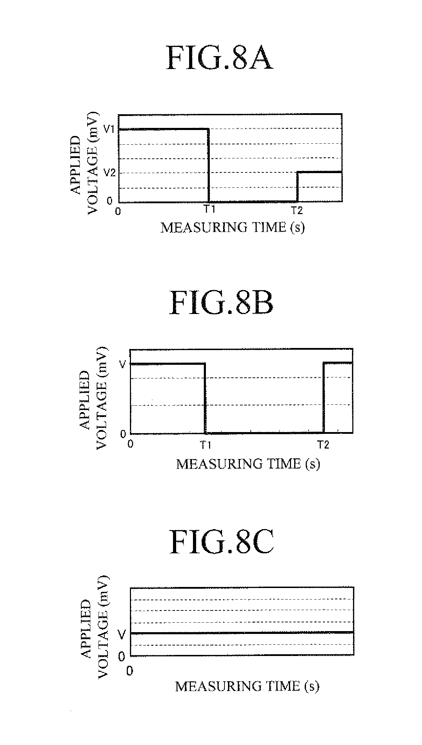

On the other hand, the voltage applied between the working electrode 24 and the counter electrode 25 is, for example, 0.1 to 1.0 volt. A pattern of the voltage applied between the working electrode 24 and the counter electrode 25 is selected, for example, from patterns shown in Figs. 8A, 8B and 8C, taking a time point when it is verified that the working electrode 24 is communicated with the counter electrode 25 through liquid as 0 seconds. In a voltage application pattern shown in Fig. 8A, a certain voltage V1 is applied for a certain period of time T1 from the time point of verifying communication through liquid, and then the application of a voltage is stopped for a certain period of time (T2-T1), and further a certain voltage V2 which is smaller than V1 is applied. In a voltage application pattern shown in Fig. 8B, a certain voltage V is applied for a certain period of time T1 from the time point of verifying communication through liquid, and then the application of a voltage is stopped for a certain period of time (T2-T1), and further a certain voltage V which is equal to the initial voltage is applied. In a voltage application pattern shown in Fig. 8C, a certain voltage V is continuously applied from the time point of verifying communication through liquid.

Next, the calculating part 13 determines a peak value or an end value of the response current (S3), and calculates a relative value for the peak value or the end value (S4). In this case, in the voltage application patterns shown in Figs. 8A and 8B, a peak value appears in both periods which are a period before time T1 and a period after time T2, but any peak value may be employed in S3 as long as the sample can be distinguished from the control liquid. Further, an end value refers to a final response current value in a predetermined measuring time of a response current.

The calculating part 13 further calculates a change rate of the previously calculated relative value (S5) and the control part 14 distinguishes between a sample and a control liquid based on the change rate (S6).

The control part 14 determines a liquid supplied to the capillary 23 to be a sample, for example, when a summed value of change rate values at a plurality of specific times is a predetermined threshold value or more, and determines the liquid to be a control liquid when the summed value of change rate values is smaller than the threshold value. In this case, the summed value of change rate values and the threshold value may be determined in accordance with the species of the sample, the species of the specific component to be analyzed, the composition of the control liquid and the voltage application pattern. For example, when glucose in blood is analyzed by applying a certain voltage continuously from the time point of communication through liquid, the control part 14 is configured so as to determine a liquid to be a sample when a summed value of change rate values at lapses of 3.8 seconds, 4.0 seconds, and 4.2 seconds from the time point of communication through liquid is "-3" or more, and determine a liquid to be a control liquid when the summed value of change rate values is less than "-3".

The control part 14 may be configured so as to determine an object to be a sample when a summed value of differences between a change rate value at a first specific time and change rate values at a plurality of second specific times is a certain value or more, and determine an object to be a control liquid when the summed value of the differences is smaller than the certain value. For example, when glucose in blood is analyzed by applying a certain voltage continuously from the time point of communication through liquid, the control part 14 may be configured so as to determine a liquid to be a sample when a summed value of a difference between change rate values at lapses of 3.0 seconds and 2.2 seconds from the time point of communication through liquid, a difference between change rate values at lapses of 3.0 seconds and 2.4 seconds, a difference between change rate values at lapses of 3.0 seconds and 2.6 seconds, and a difference between change rate values at lapses of 3.0 seconds and 2.8 seconds is "0.2" or more, and determine a liquid to be a control liquid when the summed value of differences is less than "0.2".

The control part 14 may be further configured so as to determine an object to be a control liquid when an average value of change rate values at a plurality of specific times is a certain value or more, and determine an object to be a sample when the average value is smaller than the certain value. For example, when glucose in blood is analyzed by applying a certain voltage continuously from the time point of communication through liquid, and a liquid containing mannose is used as a control liquid, the control part 14 may be configured so as to determine a liquid to be a control liquid when an average value of change rate values between a lapse of 1.0 second and a lapse of 2.0 seconds from the time point of communication through liquid is "-0.0175" or more, and determine a liquid to be a sample when the average value of change rate values is less than "-0.0175".

When the control part 14 determines that the liquid is a sample (S6: Yes), a specific component in the sample is analyzed (S7), and when the control part 14 determines that the liquid is a control liquid (S6: No), a state of the analyzing apparatus 1 is inspected using the control liquid (S8). This inspection is performed in the same manner as in a normal sample analysis. For example, when a specific component at the time of analyzing a control liquid is within a prescribed range, the control part 14 determines that the analyzing apparatus 1 is operated normally, and when a concentration of the specific component is not within a prescribed range, the control part 14 determines that the analyzing apparatus 1 is abnormal.

In the analyzing apparatus 1, the sample such as a whole blood is automatically distinguished from the control liquid. Therefore, when measuring the control liquid, a user does not need to select a mode for measuring a control liquid and a burden of the user is reduced. Further, if the control liquid is automatically distinguished, there does not arise a situation where the inspection of the analyzing apparatus 1 is performed without changing a mode from a normal measurement mode to a control liquid measurement mode, and conversely the measurement of the sample is performed without changing a mode from a control liquid measurement mode to a normal measurement mode. As a result, accurate inspection results or measurement results can be obtained and the need for reinspecting or remeasuring does not arise.

The present invention is not limited to the embodiments described above. For example, configurations of the analyzing apparatus 1 and the biosensor 2 are not limited to the illustrated configurations.

### Example 1

In this example, whether or not a blood sample and a control liquid can be distinguished based on the change rate of the response current in a blood sugar level measurement system using a conventional blood sugar level measurement apparatus and a biosensor was studied.

"GT-1810" (manufactured by ARKRAY, Inc.) was used as a blood sugar level measurement apparatus, and "G Sensor" (manufactured by ARKRAY, Inc.) was used as a biosensor.

Nine kinds of the whole bloods having different hematocrit levels (Hct) and glucose concentrations were used as a blood sample. Each of the hematocrit levels was set at 20%, 42% and 60%, and each of the glucose concentrations was set at 60 mg/dL, 120 mg/dL and 320 mg/dL. Three samples for each kind of blood sample, totally 27 samples, were used. Solutions having the composition shown in the following Table 1 were used as a control liquid.

**[Table 1]**

| Components | Composition | | |
|---|---|---|---|
| D-glucose | 500mg/dL (Low concentration) | 1305mg/dL (Medium concentration) | 2997mg/dL ( High concentration) |
| Food Red No.40 | 400mg | | |
| ECHO GUM T | 1250mg | | |
| Priclin 300 | 1mL | | |
| Bis Tris | 10.46g | | |
| Purified water | 1000mL | | |

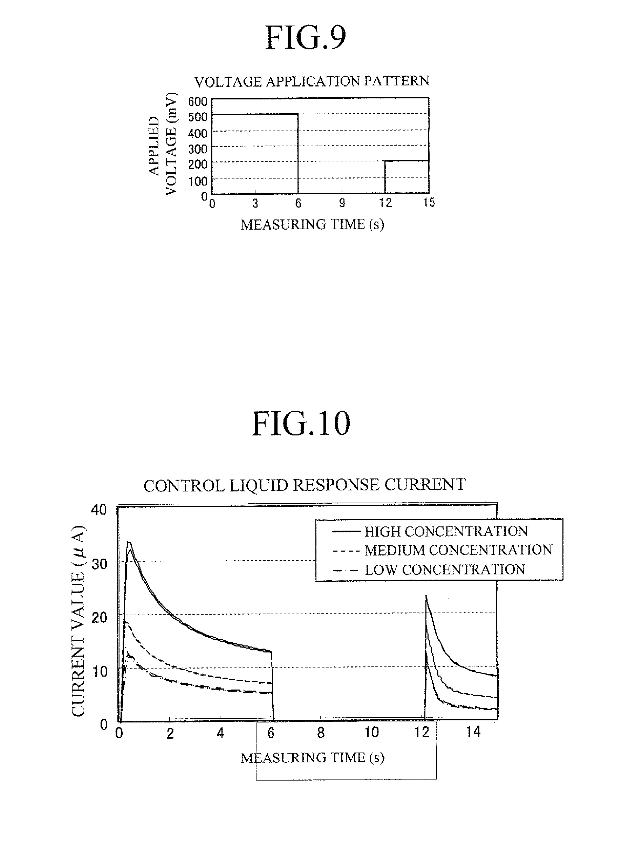

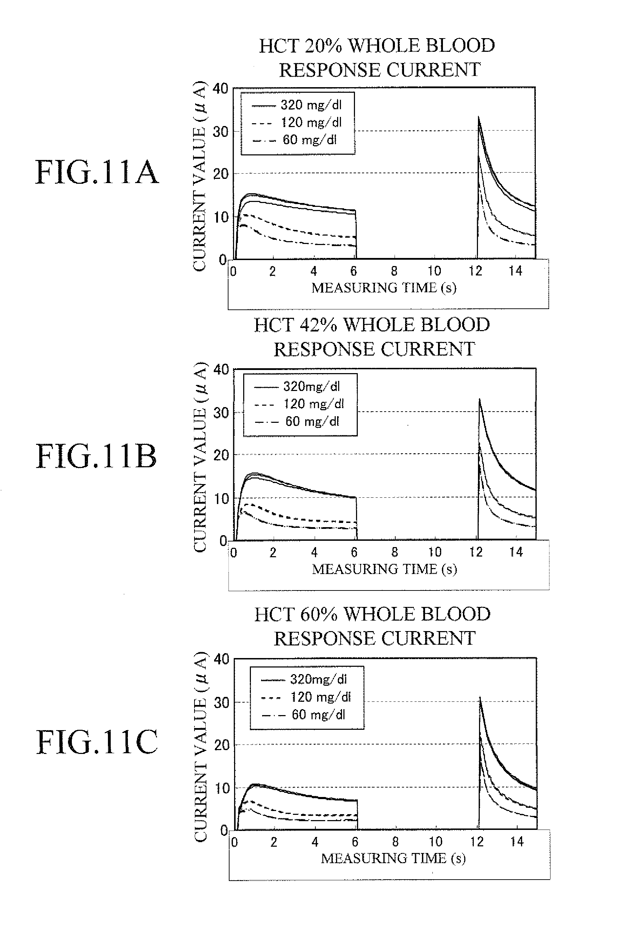

A response current was measured by applying a voltage in a pattern shown in Fig. 9 between the working electrode and the counter electrode of the biosensor. The measurement results of the response current of the control liquid are shown in Fig. 10, and those of the whole blood are shown in Figs. 11A, 11B and 11C.

As is apparent from comparisons between Fig. 10 and Figs. 11A, 11B,11C, it was difficult to distinguish all control liquids of low concentration, medium concentration and high concentration from the whole bloods having various hematocrit levels (Hct) and glucose concentrations only by the response current.

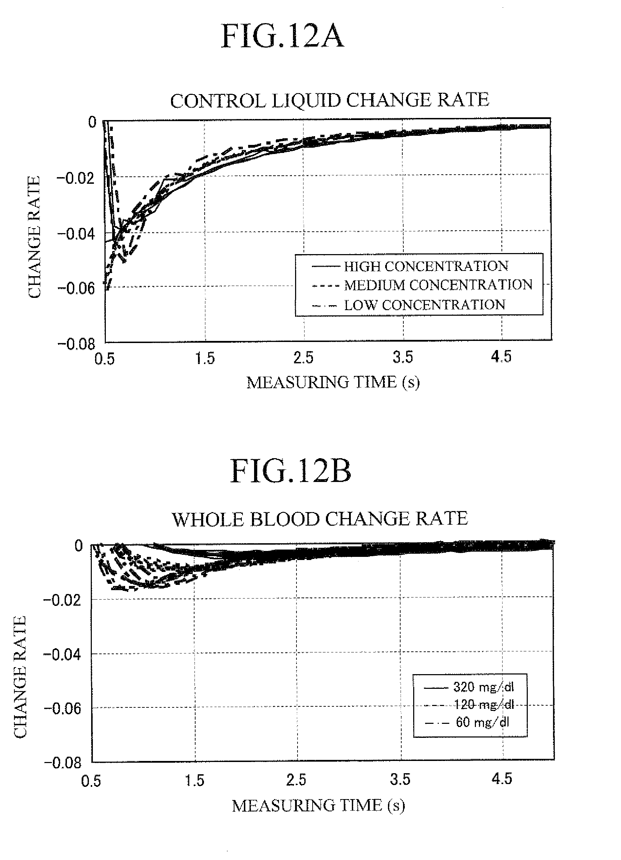

Next, a relative value for a peak value of the response current was calculated, and a change rate of the relative value was calculated. The calculation results of the change rate are shown in Figs. 12A and 12B.

As is apparent from comparisons between Fig. 12A and Fig. 12B, the change rates of the relative value for the peak value of the response current were different between the control liquid and the whole blood. Particularly, in a range between measuring times of 0.5 seconds and 1.0 second of the response current, a difference in change rates between the control liquid and the whole blood was large. Therefore, values of the change rate at 0.5 seconds, 0.6 seconds, 0.7 seconds, 0.8 seconds, 0.9 seconds and 1.0 second were summed up for both the control liquid and the whole blood, and the summed values were compared between the control liquid and the whole blood, and consequently the probability of distinguishing the control liquid from the whole blood was 100% if setting the threshold at "-0.1".

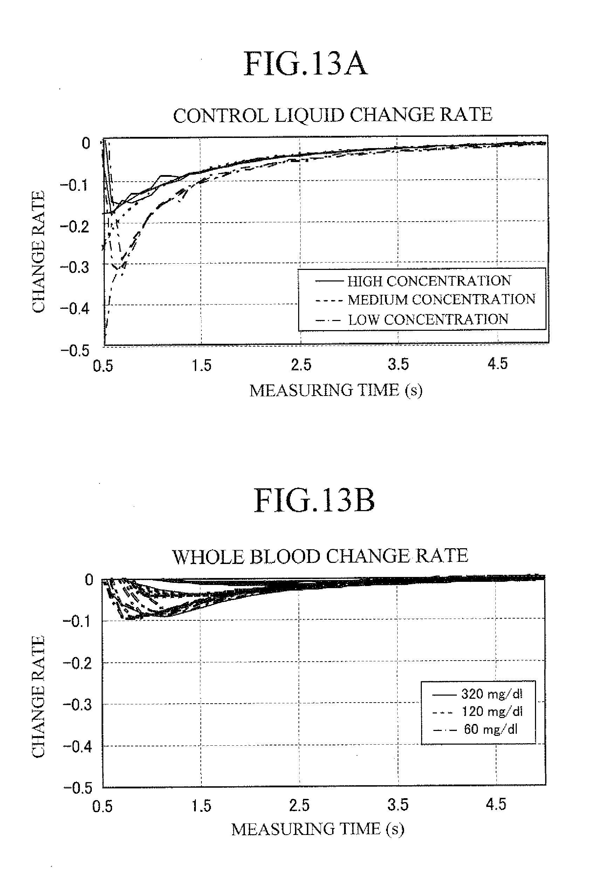

Next, a relative value for an end value (a response current value at the time when measuring time is 15 seconds) of the response current was calculated, and a change rate of the relative value was calculated. The calculation results of the change rate are shown in Figs. 13A and 13B.

As is apparent from comparisons between Fig. 13A and Fig. 13B, the change rates of the relative value for the end value of the response current were different between the control liquid and the whole blood. Particularly, in a range between measuring times of 0.5 seconds and 1.0 second of the response current, a difference in change rates between the control liquid and the whole blood was large. Therefore, values of the change rate at 0.5 seconds, 0.6 seconds, 0.7 seconds, 0.8 seconds, 0.9 seconds and 1.0 second were summed up for both the control liquid and the whole blood, and the summed values were compared between the control liquid and the whole blood, and consequently the probability of distinguishing the control liquid from the whole blood was 100% if setting the threshold at "-0.5".

### Example 2

In this example, whether or not a blood sample and a control liquid can be distinguished based on the change rate of the response current in a blood sugar level measurement system using a conventional blood sugar level measurement apparatus and biosensor was studied in the same manner as in Example 1.

"GT 1641/61" (manufactured by ARKRAY, Inc.) was used as a blood sugar level measurement apparatus, and "Dia SENSOR" (manufactured by ARKRAY, Inc.) was used as a biosensor.

Whole blood samples (9 kinds, 27 samples) similar to those in Example 1 were used as a blood sample, and solutions having the composition shown in the following Table 2 were used as a control liquid.

**[Table 2]**

| Components | Composition | | |
|---|---|---|---|
| D-glucose | 600mg/dL (Low concentration) | 1080mg/dL (Medium concentration) | 2860mg/dL( High concentration) |
| Food Red No. 106 | 100mg | | |
| Benzoic acid | 1000mg | | |
| Purified water | 1000mL | | |

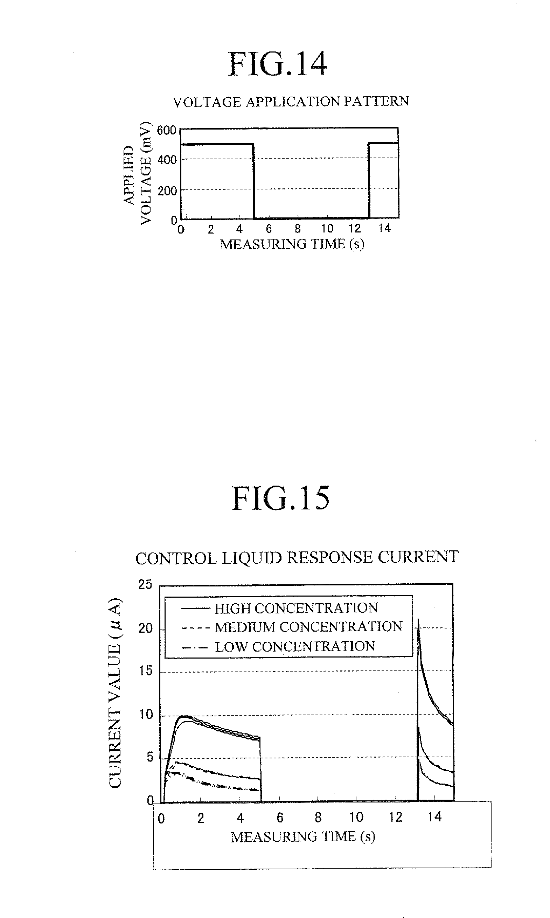

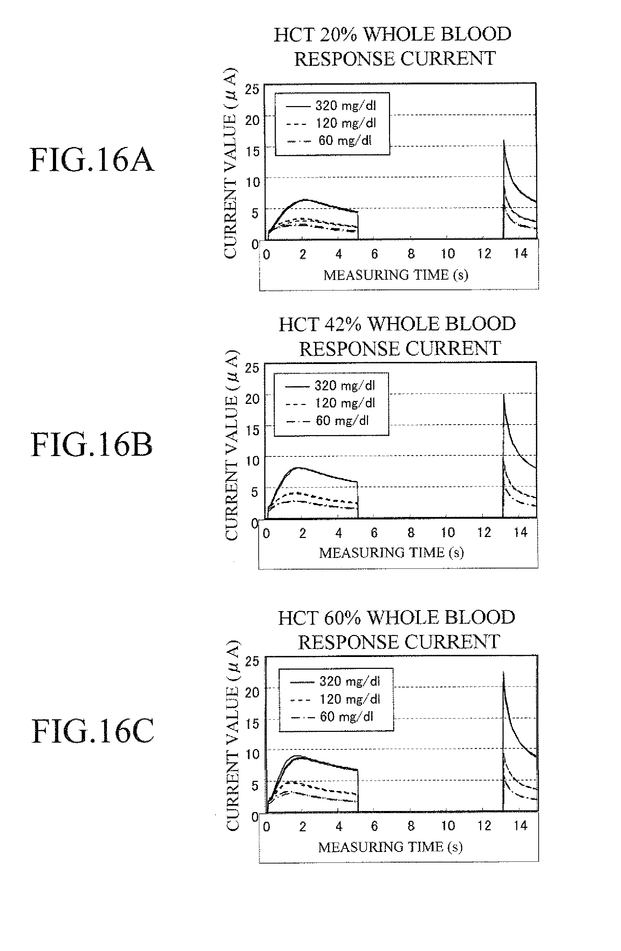

A response current was measured by applying a voltage in a pattern shown in Fig. 14 between the working electrode and the counter electrode of the biosensor. The measurement results of the response current of the control liquid are shown in Fig. 15, and those of the whole blood are shown in Figs. 16A, 16B and 16C.

As is apparent from comparisons between Fig. 15 and Figs. 16A, 16B, 16C, it was difficult to distinguish all control liquids of low concentration, medium concentration and high concentration from the whole bloods having various hematocrit levels (Hct) and glucose concentrations only by the response current.

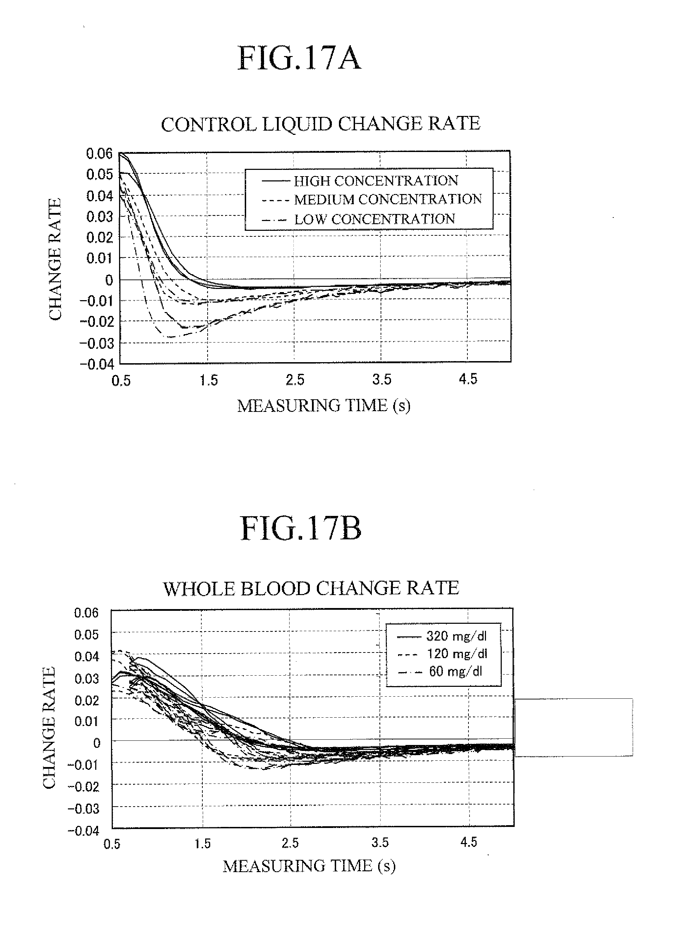

Next, a relative value for a peak value of the response current was calculated, and a change rate of the relative value was calculated. As the peak value, a peak value of the response current at the time of applying a voltage was employed. The calculation results of the change rate are shown in Figs. 17A and 17B.

As is apparent from comparisons between Fig. 17A and Fig. 17B, the change rates of the relative value for the peak value of the response current were different between the control liquid and the whole blood. Particularly, in a range between measuring times of 0.5 seconds and 1.0 second of the response current, a difference in change rates between the control liquid and the whole blood was large. Therefore, a difference between values of the change rate at 1 second and at 0.5 seconds, a difference between values of the change rate at 1 second and at 0.6 seconds, a difference between values of the change rate at 1 second and at 0.7 seconds, a difference between values of the change rate at 1 second and at 0.8 seconds, and a difference between values of the change rate at 1 second and at 0.9 seconds were summed up for both the control liquid and the whole blood, and the summed values were compared between the control liquid and the whole blood, and consequently the probability of distinguishing the control liquid from the whole blood was 100% if setting the threshold at "-0.1".

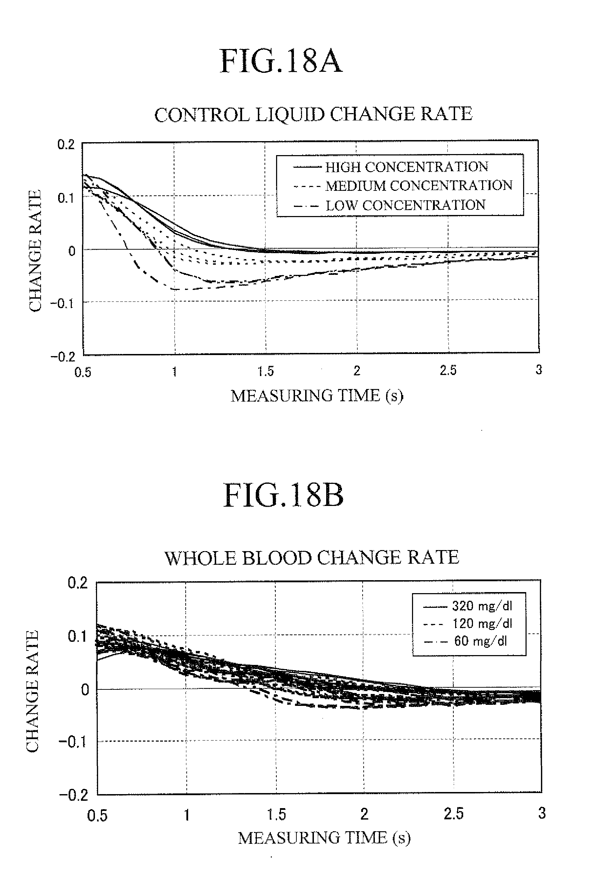

Next, a relative value for an end value (a response current value at the time when a measuring time is 15 seconds) of the response current was calculated, and a change rate of the relative value was calculated. The calculation results of the change rate are shown in Figs. 18A and 18B.

As is apparent from comparisons between Fig. 18A and Fig. 18B, the change rates of the relative value for the end value of the response current were different between the control liquid and the whole blood. Particularly, in a range between measuring times of 0.5 seconds and 1.0 second of the response current, a difference in change rates between the control liquid and the whole blood was large. Therefore, a difference between values of the change rate at 1 second and at 0.5 seconds, a difference between values of the change rate at 1 second and at 0.6 seconds, a difference between values of the change rate at 1 second and at 0.7 seconds, a difference between values of the change rate at 1 second and at 0.8 seconds, and a difference between values of the change rate at 1 second and at 0.9 seconds were summed up for both the control liquid and the whole blood, and the summed values were compared between the control liquid and the whole blood, and consequently the probability of distinguishing the control liquid from the whole blood was 100% if setting the threshold at "-0.24". Example 3

In this example, whether or not a blood sample and a control liquid can be distinguished based on the change rate of the response current in a blood sugar level measurement system using a conventional blood sugar level measurement apparatus and biosensor was studied in the same manner as in Example 1.

"GT 1910" (manufactured by ARKRAY, Inc.) was used as a blood sugar level measurement apparatus, and "Glucocard X-SENSOR" (manufactured by ARKRAY, Inc.) was used as a biosensor.

Whole blood samples (9 kinds, 27 samples) similar to those in Example 1 were used as a blood sample, and solutions having the composition shown in the following Table 3 were used as a control liquid.

**[Table 3]**

| Components | Composition | | |
|---|---|---|---|
| D-glucose | 470mg/dL (Low concentration) | 1110mg/dL (Medium concentration) | 2400mg/dL ( High concentration) |
| Food Blue No. 1 | 2000mg | | |
| PVA 1000 | 30.0mg | | |
| Priclin 300 | 1mL | | |
| MES | 10.663 g | | |
| Purified water | 1000mL | | |

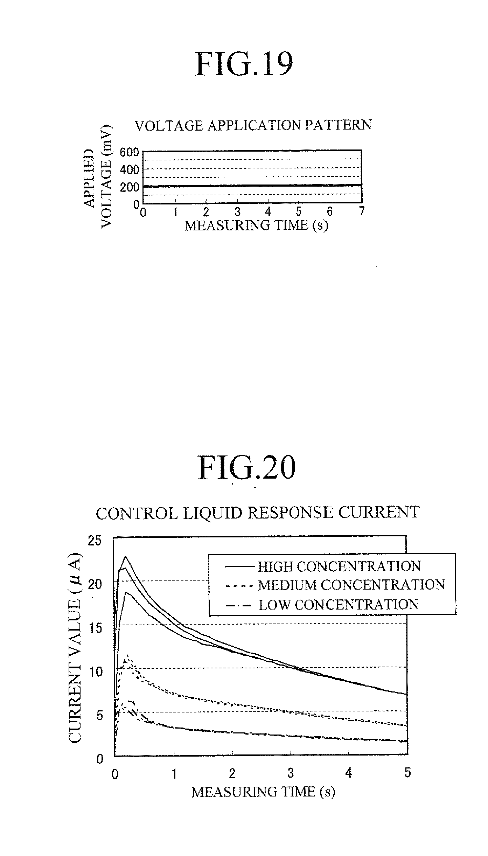

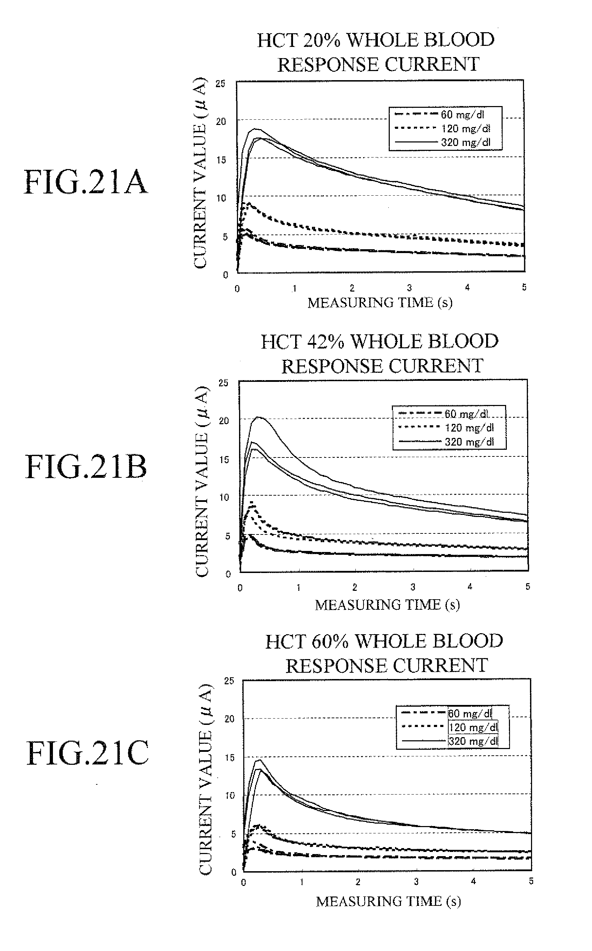

A response current was measured by applying a voltage in a pattern shown in Fig. 19 between the working electrode and the counter electrode of the biosensor. The measurement results of the response current of the control liquid are shown in Fig. 20, and those of the whole blood are shown in Figs. 21A, 21B and 21C.

As is apparent from comparisons between Fig. 20 and Fig. 21, it was difficult to distinguish all control liquids of low concentration, medium concentration and high concentration from the whole bloods having various hematocrit levels (Hct) and glucose concentrations only by the response current.

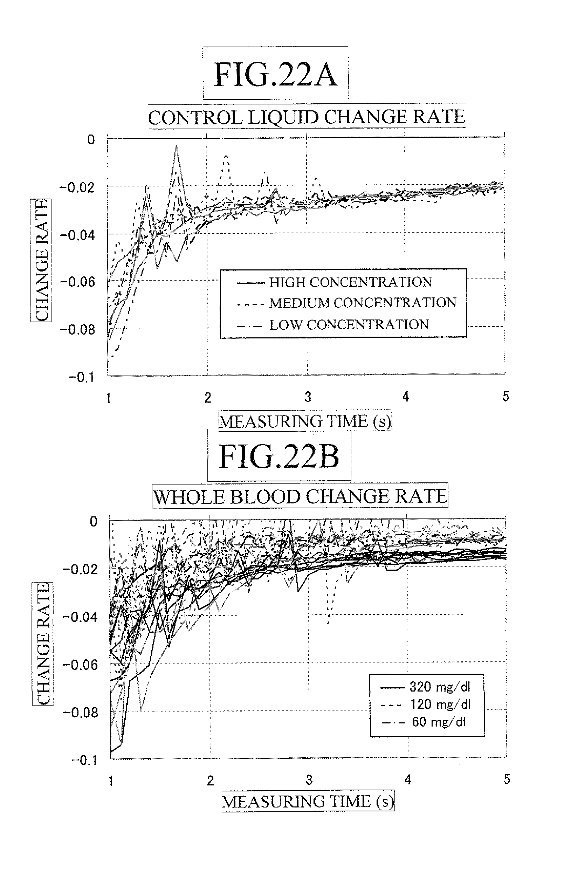

Next, a relative value for an end value (a response current value at the time when a measuring time is 5 seconds) of the response current was calculated, and a change rate of the relative value was calculated. The calculation results of the change rate are shown in Figs. 22A and 22B.

As is apparent from comparisons between Fig. 22A and Fig. 22B, the change rates of the relative value for the end value of the response current were different between the control liquid and the whole blood. Particularly, in a range between a measuring time of 3.4 seconds and 5.0 seconds of the response current, a difference in change rates between the control liquid and the whole blood was large. Therefore, values of the change rate at 3.4 seconds, 3.6 seconds, 3.8 seconds, 4.0 seconds, 4.2 seconds, 4.4 seconds, 4.6 seconds, 4.8 seconds, and 5.0 seconds were summed up for both the control liquid and the whole blood, and the summed values were compared between the control liquid and the whole blood, and consequently the probability of distinguishing the control liquid from the whole blood was 100% if setting the threshold at "-0.18".

### Example 4

In this example, an effect of species of the additive added to the control liquid on a distinction between a control liquid and a sample based on the change rate of the response current was studied.

Taking the composition shown in the above Table 3 as basic composition (without additive), solutions formed by adding mannose, xylose or galactose as an additive to a liquid having this basic composition were used as a control liquid. An addition amount of the additive was 4.0 M. Whole blood samples (9 kinds, 27 samples) similar to those in Example 1 were used as a blood sample.

A response current was measured, using "GT 1910" (manufactured by ARKRAY, Inc.) as a blood sugar level measurement apparatus, and using "Glucocard X-SENSOR" (manufactured by ARKRAY, Inc.) as a biosensor.
An application pattern of a voltage applied between the working electrode and the counter electrode of the biosensor was as shown in Fig. 19.

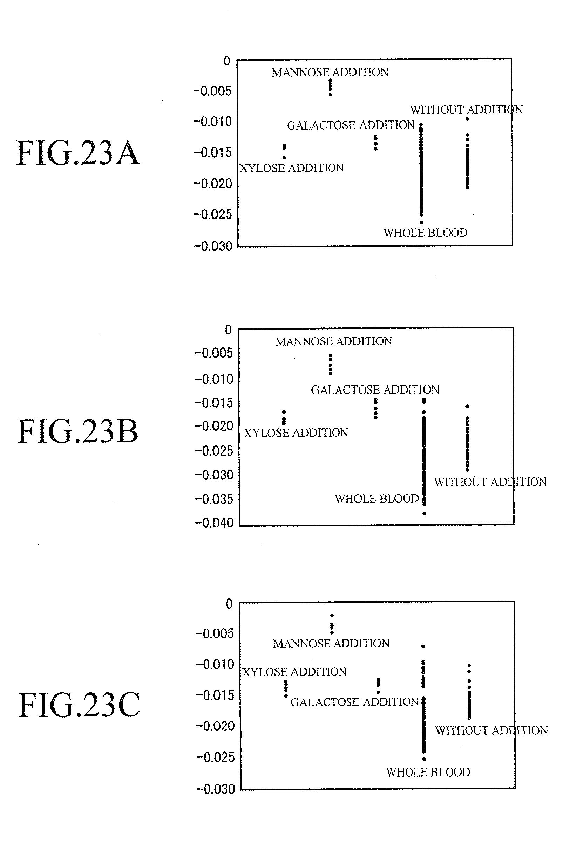

On the other hand, the change rate of the relative value for the peak value (maximum value) of the response current was calculated based on the measured response current values. The change rates of the relative value are shown in terms of average between 1.4 seconds and 2.4 seconds in Fig. 23A, average between 1.0 second and 2.0 seconds in Fig. 23B, and average between 1.8 seconds and 2.2 seconds in Fig. 23C.

As is apparent from Figs. 23A, 23B and 23C, in the average of the change rates in a certain range of time, a difference between the control liquid and the whole blood becomes clear in the case of using mannose as an additive. Therefore, it becomes possible to distinguish between the control liquid and the whole blood by adding an appropriate substance such as mannose to the control liquid and calculating the average of the change rates in a certain range of time.

As is apparent from the above, in accordance with the change rate of the relative value for the peak value of the response current value, it is possible to appropriately distinguish between the control liquid and the whole blood even in the case of various combinations of blood sugar level measurement apparatus and biosensor by properly setting a computing equation and a threshold, using a range where a difference in change rates between the control liquid and the whole blood is large. Moreover, it can be seen that the control liquid can be more appropriately distinguished from the whole blood by adding an additive such as mannose to the control liquid.

## Claims

1. A control liquid discriminating method for distinguishing between a sample and a control liquid in a system for analyzing a specific component in the sample by using an analyzing tool having a working electrode and an counter electrode, wherein the method comprises:
a first step of applying a voltage between the working electrode and the counter electrode;
a second step of measuring a response current at certain intervals by use of the working electrode and the counter electrode;
a third step of calculating a relative value for a peak value or an end value of the response current;
a fourth step of calculating a change rate of the relative value; and
a fifth step of distinguishing between the sample and the control liquid based on the change rate.

2. The control liquid discriminating method according to claim 1, wherein in the fifth step, an object is determined to be a sample when a summed value of change rate values at a plurality of specific times is a certain value or more, and determined to be a control liquid when the summed value of change rate values is smaller than the certain value.

3. The control liquid discriminating method according to claim 1, wherein in the fifth step, an object is determined to be a sample when a summed value of differences between a change rate value at a first specific time and change rate values at a plurality of second specific times is a certain value or more, and determined to be a control liquid when the summed value of differences is smaller than the certain value.

4. The control liquid discriminating method according to claim 1, wherein in the fifth step, an object is determined to be a control liquid when an average value of change rate values at a plurality of specific times is a certain value or more, and determined to be a sample when the average value is smaller than the certain value.

5. The control liquid discriminating method according to claim 4, wherein a liquid containing mannose is used as the control liquid.

6. The control liquid discriminating method according to claim 1, wherein
the first step is performed by continuously applying a certain voltage between the working electrode and the counter electrode, and
in the third step, a calculation is performed, taking a maximum value of the response current obtained in the second step as a peak value.

7. The control liquid discriminating method according to claim 1, wherein
the first step is performed by applying a certain voltage between the working electrode and the counter electrode for a certain period of time, then stopping the application of a voltage for a certain period of time, and further applying a certain voltage between the working electrode and the counter electrode, and
in the third step, a calculation is performed using a peak value selected from a plurality of peak values of the response current obtained in the second step.

8. The control liquid discriminating method according to claim 1, wherein the first step is performed by continuously applying a certain voltage between the working electrode and the counter electrode, and
in the third step, a calculation is performed using an end value of the response current obtained in the second step.

9. The control liquid discriminating method according to claim 1, wherein
the first step is performed by applying a certain voltage between the working electrode and the counter electrode for a certain period of time, then stopping the application of a voltage for a certain period of time, and further applying a certain voltage between the working electrode and the counter electrode, and
in the third step, a calculation is performed using an end value of the response current obtained in the second step.

10. The control liquid discriminating method according to claim 1, wherein the sample is a whole blood and the specific component is glucose.

11. An analyzing apparatus for analyzing a specific component in a sample by using an analyzing tool having a working electrode and an counter electrode, wherein the analyzing apparatus comprises:
voltage application means for applying a voltage between the working electrode and the counter electrode;
current measuring means for measuring a response current at certain intervals when a voltage is applied between the working electrode and the counter electrode;
calculating means for calculating a relative value for a peak value or an end value of the response current and for calculating a change rate of the relative value; and
control means for distinguishing between a sample and a control liquid based on the change rate calculated in the calculating means.

12. The analyzing apparatus according to claim 11, wherein the control means is configured so as to determine an object to be a sample when a summed value of change rate values at a plurality of specific times is a certain value or more, and determine an object to be a control liquid when the summed value of change rate values is smaller than the certain value.

13. The analyzing apparatus according to claim 11, wherein the control means is configured so as to determine an object to be a sample when a summed value of differences between a change rate value at a first specific time and change rate values at a plurality of second specific times is a certain value or more, and determine an object to be a control liquid when the summed value of differences is smaller than the certain value.

14. The analyzing apparatus according to claim 11, wherein the control means is configured so as to determine an object to be a control liquid when an average value of change rate values at a plurality of specific times is a certain value or more, and determine an object to be a sample when the average value is smaller than the certain value.

15. The analyzing apparatus according to claim 11, wherein
the control means is configured so as to control the voltage application means in order to continuously apply a certain voltage between the working electrode and the counter electrode, and
the calculating means is configured so as to calculate the change rate, taking a maximum value of the response current measured in the current measuring means as a peak value.

16. The analyzing apparatus according to claim 11, wherein
the control means is configured so as to control the voltage application means in order to apply a certain voltage between the working electrode and the counter electrode for a certain period of time, then stop the application of a voltage for a certain period of time, and further apply a certain voltage between the working electrode and the counter electrode, and
the calculating means is configured so as to calculate the change rate using a peak value selected from a plurality of peak values of the response current measured in the current measuring means.

17. The analyzing apparatus according to claim 11, wherein
the control means is configured so as to control the voltage application means in order to continuously apply a certain voltage between the working electrode and the counter electrode, and
the calculating means is configured so as to calculate the change rate, using an end value of the response current measured in the current measuring means.

18. The analyzing apparatus according to claim 11, wherein
the control means is configured so as to control the voltage application means in order to apply a certain voltage between the working electrode and the counter electrode for a certain period of time, then stop the application of a voltage for a certain period of time, and further apply a certain voltage between the working electrode and the counter electrode, and
the calculating means is configured so as to calculate the change rate using an end value of the response current measured in the current measuring means.

19. The analyzing apparatus according to claim 11, which is configured so as to analyze glucose as the specific component in a whole blood as the sample.
